# DEMANDE DE BREVET EUROPEEN

(11) **EP 1 438 949 A1**
(43) Date de publication de la demande: **21.07.2004**
(21) Numéro de dépôt: 04290072.0
(22) Date de dépôt: 12.01.2004
(51) Int. Cl.: A61K 7/13

(54) **Composition pour la teinture des fibres kératiniques comprenant un coupleur acylaminophénol**

(30) Priorité: 17.01.2003 FR 0300540
(71) Demandeur: L'OREAL, 75008 Paris (FR)
(72) Inventeur: Vidal, Laurent, 75013 Paris (FR); Lagrange, Alain, 77700 Coupvray (FR)
(74) Mandataire: Wattremez, Catherine

(57) **Abrégé**

L'invention a pour objet une composition tinctoriale comprenant un coupleur acylaminophénol particulier et utilisation de cette composition pour la teinture des fibres kératiniques, en particulier les cheveux.

La présente invention permet en particulier d'obtenir une coloration de fibres kératiniques chromatique, puissante, peu sélective et tenace.

## Description

L'invention a pour objet une nouvelle composition tinctoriale comprenant un coupleur acylaminophénol particulier, l'utilisation de cette composition pour la teinture des fibres kératiniques ainsi que le procédé de teinture de fibres kératiniques à partir de cette composition.

Il est connu de teindre les fibres kératiniques et en particulier les cheveux humains avec des compositions tinctoriales contenant des précurseurs de colorant d'oxydation, en particulier des ortho ou para-phénylènediamines, des ortho ou para-aminophénols, des composés hétérocycliques tels que des dérivés de diaminopyrazole, des dérivés de pyrazolo[1,5-a]pyrimidine, des dérivés de pyrimidines, des dérivés de pyridine, des dérivés de 5,6-dihydroxyindole, des dérivés de 5,6-dihydroxyindoline appelés généralement bases d'oxydation. Les précurseurs de colorants d'oxydation ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les méta-diamines aromatiques, les méta-aminophénols, les méta-hydroxyphénols et certains composés hétérocycliques tels que par exemple des dérivés de pyrazolo[1,5-b]-1,2,4,-triazoles, des dérivés de pyrazolo[3,2-c]-1,2,4,-triazoles, des dérivés de pyrazolo[1,5-a]pyrimidines, des dérivés de pyridine, des dérivés de pyrazol-5-one, des dérivés d'indoline et des dérivés d'indole.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée, présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possibles, c'est à dire permettre d'obtenir des écarts de coloration les plus faibles possibles tout au long d'une même fibre kératinique, qui peut être en effet différemment sensibilisée (i.e. abîmée) entre sa pointe et sa racine. Ils doivent également présenter une bonne stabilité chimique dans les formulations. Ils doivent présenter un bon profil toxicologique.

Il a déjà été proposé, notamment dans les demandes de brevet WO 98/52519, JP2521636, FR-A-1 596 879 ou encore FR-A-2 233 984, des compositions de teinture d'oxydation contenant certains dérivés de N-(2-hydroxy-4-amino-phényl)-acétamide à titre de coupleurs, en association avec des bases d'oxydation classiquement utilisées en teinture d'oxydation telles que par exemple des paraphénylènediamines, des para-aminophénols ou bien encore certaines pyridines.

De telles compositions ne sont cependant pas toujours satisfaisantes, notamment du point de vue de la puissance des colorations obtenues. De plus, les couleurs obtenues sur les cheveux par association de tels coupleurs avec des dérivés de para-phénylènediamine ne sont pas stables sous l'action de la lumière et présentent un virage de couleur inesthétique.

Le but de la présente invention est de développer de nouvelles compositions tinctoriales ne présentant pas les inconvénients des compositions de la technique antérieure. En particulier, le but de l'invention est de développer de nouveaux coupleurs présentant des propriétés telles que les compositions les contenant permettent de conférer aux cheveux d'excellentes propriétés d'intensité de couleur, de variété de nuances, d'uniformité de la couleur et de ténacité vis à vis des différentes agressions extérieures que peuvent subir les cheveux.

Ce but est atteint avec la présente invention qui a pour objet une composition tinctoriale comprenant, dans un milieu cosmétique approprié à la teinture des fibres kératiniques, au moins une base d'oxydation et au moins un coupleur de formule (1) suivante et/ou un de ces sels d'addition avec une base ou un acide 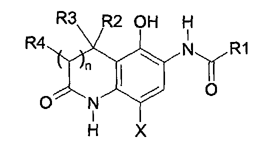 dans laquelle
- **R**_{**1**} est un radical alkyle comprenant de 1 à 10 atomes de carbone, de préférence de 1 à 6 atomes de carbone, le radical alkyle étant éventuellement substitué par un ou plusieurs radicaux choisis parmi les atomes d'halogène, les radicaux hydroxy, alcoxy, carboxy, alkyl(C₁-C₄)carboxamido, sulfonamido, alkyl(C₁-C₄)sulfonamido, NR₁₁R₁₂, R₁₁ et R₁₂, représentant indépendamment un atome d'hydrogène, un radical (C₁-C₂)alkyle pouvant être substitué par un hydroxy ou un (C₁-C₂)alcoxy;
- **R**_{**2**}, **R**_{**3**} et **R**_{**4**} représentent indépendamment
   (i) un atome d'hydrogène,
   (ii) un radical (C₁-C₄)alkyle éventuellement substitué par un ou plusieurs radicaux choisis parmi les radicaux hydroxy, alcoxy, carboxy, alkyl(C₁-C₄)carboxamido (AlkNHCO-), alkyl(C₁-C₄)sulfonyle (AlkSO₂-), alkyl(C₁-C₄)sulfoxyde (AlkylSO-), sulfonamido (NH2-SO2-), alkyl(C₁-C₄)sulfonamido (AlkylNHSO2-) ou sulfonique ( -SO₃H) ou NR₁₁R₁₂.
- **X** représente un atome d'hydrogène, un atome d'halogène, un radical (C₁-C₄)alcoxy radical, un radical aryloxy radical, ces radicaux pouvant être substitués, et
- **n** est 0 ou 1, de préférence 1.

L'invention a aussi pour objet l'utilisation des coupleurs de formule (1) pour la teinture de fibres kératiniques, en particulier des cheveux et le procédé de teinture de fibres kératiniques.

Un autre objet de l'invention est un procédé de teinture, un kit de teinture ainsi qu'un dispositif de teinture des fibres kératiniques à partir de la composition de la présente invention.

Dans le cadre de l'invention, on entend par alkyle des radicaux linéaires ou ramifiés.

Dans la formule (I) ci dessus, R₁ peut représenter un radical méthyle, éthyle, isopropyle, méthoxyméthyle, hydroxyméthyle, 1-carboxyméthyle, 1-aminométhyl, 2-carboxyéthyle, 2-hydroxyéthyle, 3-hydroxypropyle, 1,2-dihydroxyéthyle, 1-hydroxy-2-aminoéthyle, 2-hydroxy-1-aminoéthyle.

De préférence, R₁ est choisi parmi les radicaux méthyle, éthyle, hydroxyméthyle, 2-hydroxyéthyle, 1,2-dihydroxyéthyle. Selon un mode de réalisation préféré, R₁ est choisi parmi un radical méthyle, éthyle, propyle, hydroxyméthyle, 2-hydroxyéthyle.

Selon un mode de réalisation préféré, R₁ représente un radical alkyle non substitué, par exemple méthyle, éthyle ou propyle.

Dans la formule (I) ci dessus, R₂, R₃ et R₄ représentent indépendamment de préférence un atome d'hydrogène, un radical alkyle en C₁-C₄ éventuellement substitué par un ou plusieurs radicaux choisis parmi les radicaux hydroxy, méthoxy, éthoxy, carboxy, alkyl(C1-C4)carboxamido, sulfonamido ou NR₁₁R₁₂. Selon un mode de réalisation plus préféré, R₂, R₃ et R₄ sont choisis parmi un atome d'hydrogène, un radical alkyle non substitué.

Dans la formule (I) ci dessus, lorsque X est un radical alcoxy ou aryloxy substitué, les substituants sont des radicaux classiques comme par exemple des radicaux hydroxy, amino, halogène, alcoxy, amido, etc. De préférence, X est choisi parmi un atome d'hydrogène, un atome de chlore, un radical alcoxy par exemple méthoxy.

Selon un mode de réalisation de l'invention, le coupleur correspond à la formule (I') 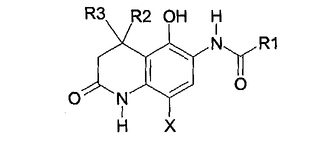 dans laquelle R₁ représente un radical alkyle en C₁-C₆, X représente un atome d'hydrogène, un atome d'halogène ou un radical alcoxy, et R₂ et R₃ représentent un radical méthyle ou éthyle.

A titre d'exemple, on peut citer les coupleurs de formule (1) ou (I') suivants :
N-(8-chloro-5-hydroxy-4,4-diméthyl-2-oxo-1,2, 3,4-tétrahydroquinolin-6-yl)-acétamide
N-(5-hydroxy-4,4-diméthyl-2-oxo-1,2,3,4-tétrahydroquinolin-6-yl)-acétamide
N-(8-chloro-5-hydroxy-4,4-diméthyl-2-oxo-1,2,3,4-tétrahydroquinolin-6-yl)-propylamide N-(5-hydroxy-4,4-diméthyl-2-oxo-1,2,3,4-tétrahydroquinolin-6-yl)-propylamide
N-(8-méthoxy-5-hydroxy-4,4-diméthyl-2-oxo-1,2,3,4-tétrahydroquinolin-6-yl)-acétamide
N-(8- méthoxy -5-hydroxy-4,4-diméthyl-2-oxo-1,2,3,4-tétrahydroquinolin-6-yl)-propylamide
N-(8-chloro-5-hydroxy-2-oxo-1,2,3,4-tétrahydroquinolin-6-yl)-acétamide
N-(5-hydroxy-2-oxo-1,2,3,4-tétrahydroquinolin-6-yl)-acétamide
N-(8-chloro-5-hydroxy-2-oxo-1,2,3,4-tétrahydroquinolin-6-yl)-propylamide
N-(5-hydroxy-2-oxo-1,2,3,4-tétrahydroquinoline-6-yl)-propylamide
N-(8-méthoxy-5-hydroxy-2-oxo-1,2,3,4-tétrahydroquinolin-6-yl)-acétamide
N-(8-méthoxy -5-hydroxy-2-oxo-1,2,3,4-tétrahydroquinolin-6-yl)-propylamide
N-(7-chloro-4-hydroxy-3,3-diméthyl-2-oxo-2,3-dihydro-indol-5-yl)-acétamide
N-(7-méthoxy-4-hydroxy-3,3-diméthyl-2-oxo-2,3-dihydro-indol-5-yl)-acétamide
N-(4-hydroxy-3,3-diméthyl-2-oxo-2,3-dihydro-indol-5-yl)-acétamide
N-(7-chloro-4-hydroxy-3,3-diméthyl-2-oxo-2,3-dihydro-indol-5-yl)-propylamide
N-(7-méthoxy-4-hydroxy-3,3-diméthyl-2-oxo-2,3-dihydro-indol-5-yl)-propylamide
N-(4-hydroxy-3,3-diméthyl-2-oxo-2,3-dihydro-indol-5-yl)-propylamide
N-(7-chloro-4-hydroxy-2-oxo-2,3-dihydro-indol-5-yl)-acétamide
N-(7-méthoxy-4-hydroxy-2-oxo-2,3-dihydroxy-indol-5-yl)-acétamide
N- (4-hydroxy-2-oxo-2,3-dihydro-indol-5-yl)-acétamide
N- (7-chloro-4-hydroxy-2-oxo-2,3-dihydro-indol-5-yl)-propylamide
N- (7-méthoxy-4-hydroxy-2-oxo-2,3-dihydro-indol-5-yl)- propylamide
N- (4-hydroxy-2-oxo-2,3-dihydro-indol-5-yl)- propylamide

De préférence, ces coupleurs sont choisis parmi le N-(8-chloro-5-hydroxy-4,4-diméthyl-2-oxo-1,2,3,4-tétrahydroquinolin-6-yl)-acétamide, le N-(5-hydroxy-4,4-diméthyl-2-oxo-1,2,3,4-tétrahydroquinolin-6-yl)-acétamide, le N-(8-chloro-5-hydroxy-4,4-diméthyl-2-oxo-1,2,3,4-tétrahydroquinolin-6-yl)-propylamide, le N-(5-hydroxy-4,4-diméthyl-2-oxo-1,2,3,4-tétrahydroquinolin-6-yl)-propylamide, le N-(8-méthoxy-5-hydroxy-4,4-diméthyl-2-oxo-1,2,3,4-tétrahydroquinolin-6-yl)-acétamideacétamide, le N-(8- méthoxy -5-hydroxy-4,4-diméthyl-2-oxo-1,2,3,4-tétrahydroquinolin-6-yl)-propylamide, le N-(8-chloro-5-hydroxy-2-oxo-1,2,3,4-tétrahydroquinolin-6-yl)-acétamide, le N-(5-hydroxy-2-oxo-1,2,3,4-tétrahydroquinolin-6-yl)-acétamide, le N-(8-chloro-5-hydroxy-2-oxo-1,2,3,4-tétrahydroquinolin-6-yl)-propylamide, le N-(5-hydroxy-2-oxo-1,2,3,4-tétrahydroquinolin-6-yl)-propylamide, le N-(8-méthoxy-5-hydroxy-2-oxo-1,2,3,4-tétrahydroquinolin-6-yl)-acétamide, le N-(8- méthoxy -5-hydroxy-2-oxo-1,2,3,4-tétrahydroquinolin-6-yl)-propylamide.

Les molécules correspondant à la formule générale (I) avec n=1 peuvent être synthétisées selon les protocoles de synthèse décrits dans le brevet US 4 564 586 et dans les références qui y sont citées.

En particulier, le 8-chloro-5-hydroxy-4,4-diméthyl-6-amino-3,4-dihydro-2(1H)-quinolinone **1** décrit dans ce brevet peut être utilisé comme réactif pour la synthèse des dérivés 6-amido de formule générale (I) par réaction classique avec le chlorure d'acide R₆-COCl dans un solvant aprotique.

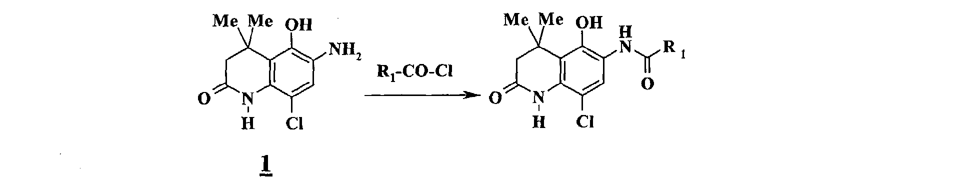

Les molécules correspondant à la formule générale (I) avec n=0 peuvent être synthétisées selon les protocoles de synthèse décrits dans le brevet US 4 904 575. En particulier, le chlorhydrate de 5-amino-7-chloro-3,3-diméthyl-4-hydroxyoxindole **2** décrit dans ce brevet peut être utilisé comme réactif pour la synthèse des dérivés 5-amido de formule générale (I) par réaction classique avec le chlorure d'acide R₆-COCl dans un solvant aprotique.

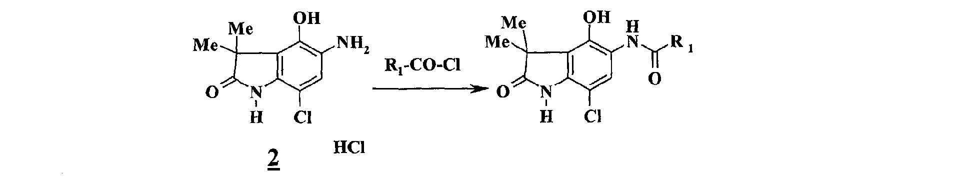

La composition tinctoriale de la présente invention comprend aussi au moins une base d'oxydation.

Cette base d'oxydation peut être n'importe quelle base d'oxydation classique dans le domaine de la coloration des fibres kératiniques. A titre d'exemple, ces bases d'oxydation sont choisies parmi les paraphénylènediamines, les bis-phénylalkylènediamines, les para-aminophénols, les bis-para-aminophénols, les ortho-aminophénols, les bases hétérocycliques et leurs sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthytoxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-p-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo(1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs d'addition avec un acide ou avec une base.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2750048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La ou les bases d'oxydation présentes dans la composition de l'invention sont en général présentent chacune en quantité comprise entre 0,001 à 10 % en poids environ du poids total de la composition tinctoriale, de préférence entre 0,005 et 6 %.

La composition tinctoriale de l'invention peut contenir en outre un ou plusieurs coupleurs additionnels conventionnellement utilisés pour la teinture de fibres kératiniques autres que ceux de formule (I). Parmi ces coupleurs, on peut notamment citer les métaphénylènediamines, les méta-aminophénols, les métadiphénols, les coupleurs naphtaléniques, les coupleurs hétérocycliques et leur sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Dans la composition de la présente invention, le ou les coupleurs sont chacun généralement présents en quantité comprise entre 0,001 et 10 % en poids environ du poids total de la composition tinctoriale, de préférence entre 0,005 et 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition tinctoriale conforme à l'invention peut en outre contenir un ou plusieurs colorants directs pouvant notamment être choisis parmi les colorants nitrés de la série benzénique, les colorants directs azoïques, les colorants directs méthiniques. Ces colorants directs peuvent être de nature non ionique, anionique ou cationique.

Le milieu cosmétique approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 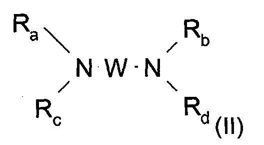 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels ou sous toute autre forme appropriée pour réaliser une teinture des fibres kératiniques, et notamment des cheveux humains.

Le procédé de la présente invention est un procédé dans lequel on applique sur les fibres la composition selon la présente invention telle que définie précédemment, et on révèle la couleur à l'aide d'un agent oxydant. La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

L'invention a aussi pour objet un kit de teinture qui comprend une première composition contenant au moins une base d'oxydation et au moins un coupleur de formule(I) et une deuxième base d'oxydation qui comprend un agent oxydant.

L'invention a enfin pour objet un dispositif à plusieurs compartiments ou "kit" de teinture dans lequel un premier compartiment renferme la composition tinctoriale de la présente invention définie ci-dessus et un deuxième compartiment renferme une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de teindre les fibres kératiniques à partir d'un procédé qui comprend le mélange d'une composition tinctoriale comprenant au moins une base d'oxydation de formule (I) avec un agent oxydant, et l'application du mélange obtenu sur les fibres kératiniques pendant un temps suffisant pour développer la coloration désirée.

Les exemples qui suivent servent à illustrer l'invention sans toutefois présenter un caractère limitatif.

### EXEMPLES

### Exemple 1 : synthèse du N- (4-hydroxy-3,3-diméthyl-2-oxo-2,3-dihydro-indol-5-yl)-acétamide

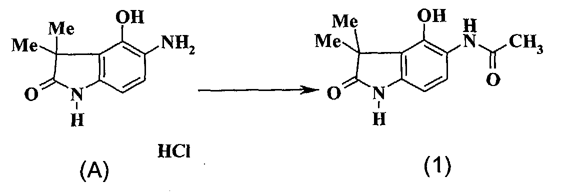

Le composé (A) est préparé selon la synthèse décrite dans le brevet US 4 904 575. En conservant la température en dessous de 5°C, 8,1 ml de chlorure d'acétyle sont ajoutés lentement à un mélange contenant du diméthylacétamide (50 ml), de l'acétate d'éthyle (50 ml) et 20 g de composé (A). Le mélange de réaction est ensuite mélangé à de l'eau glacée et agité. Le produit ainsi obtenu est filtré. Après séchage et lavage dans 100 ml d'acétate d'éthyle chaud, 23,1 g de produit (1) sont isolés par filtration et séchés.
RMN 1H (300 MHz), CDCl3) : δ 1, 30 (6H , s) 2, 10 (2H , s) 6, 33 (2H , d) 6, 89 (2H , d) 9, 73 (1H , s) 9, 94 (1H , brs) 10,20 (1H , brs)
MS m /z 234 (M+)
Point de fusion > 300 °C

### Exemple 2 : Synthèse du N- (8-chloro-4-hydroxy-3,3-diméthyl-2-oxo-2,3-dihydro-indol-5-yl)-acétamide

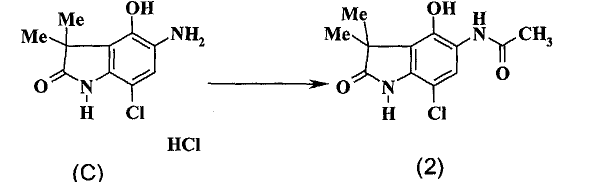

Le composé (C) est préparé selon la synthèse décrite dans le brevet US 4 904 575. En conservant la température en dessous de 5°C, 6,9 ml de chlorure d'acétyle sont ajoutés lentement à un mélange contenant du diméthylacétamide (50 ml), de l'acétate d'éthyle (50 ml) et 20 g de composé (C). Le mélange de réaction est ensuite mélangé à de l'eau glacée et agité. Le produit ainsi obtenu est filtré. Après séchage et lavage dans 100 ml d'acétate d'éthyle chaud, 22,7 g de produit (2) sont isolés par filtration et séchés.
RMN 1H (300 MHz), CDCl3) : δ 1,32 (6H , s) 2,10 (2H , s) 7,06 (1H , s) 9,75 (1H , b r s) 9, 83 (1H , s) 10,62 (1H , s)
MS m /z 268 (M+)
Point de fusion = 273-275 °C

### Exemple 3 : synthèse du N-(8-chloro-5-hydroxy-4,4-diméthyl-2-oxo-1,2,3,4-tétrahydroquinolin-6-yl)-acétamide

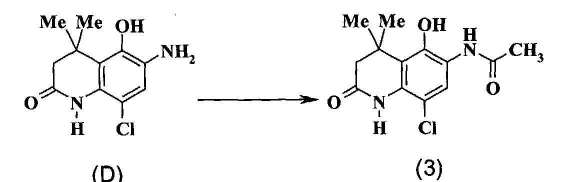

Le composé (D) est préparé selon la synthèse décrite dans le brevet US 4 430 423. En conservant la température en dessous de 5°C, 3,9 ml de chlorure d'acétyle sont ajoutés lentement à 80 ml de diméthylacétamide contenant 12 g de composé (D). Le mélange de réaction est ensuite mélangé à de l'eau glacée et agité. Le produit ainsi obtenu est filtré. Après séchage et lavage dans 60 ml d'acétate d'éthyle chaud, 13,0 g de produit (3) sont isolés par filtration et séchés.
RMN 1H (300 MHz), CDCl3) : δ 1,38 (6H , s) 2,09 (2H , s) 7, 18 (1H , s) 9, 20 (1H , b rs)9,39(1H,s)9,78(1H,brs)
MS m /z 282 (M+)
Point de fusion = 183-184 °C

### Exemple 4 : synthèse du N-(5-hydroxy-4,4-diméthyl-2-oxo-1,2,3,4-tétrahydroquinolin-6-yl)-trifluorométhylamide (4)

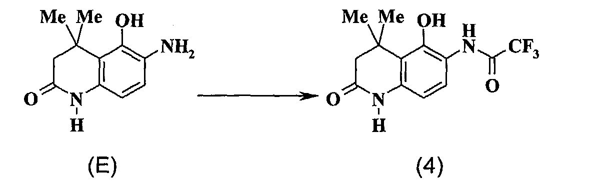

Le composé (E) est préparé selon la synthèse décrite dans le brevet US 4 430 423. 7,5 ml d'anhydride trifluoroacétique sont lentement ajoutés à une solution contenant le composé (E) dans 40 ml d'acétonitrile à température ambiante. Le mélange de réaction est mélangé à 100 ml d'acétate d'éthyle, puis lavé à l'eau. La phase organique est séchée sur du sulfate de magnésium. L'acétate d'éthyle est ensuite évaporé sous pression réduite. Après recristallisation dans 50 ml d'acétonitrile, 12,5 g du composé (4) sont obtenus et séchés.
RMN 1H (300 MHz), CDCl3) : δ 1,35 (6H , s) 2,33 (2H , s) 6,39 (2H , d) 6,90 (2H , d) 9,10 (1H, brs) 10,40 (1H, brs)
MS m /z 300 (M+)
Point de fusion = 126-129 °C

### Exemple 5 : synthèse du N-(5-hydroxy-4,4-diméthyl-2-oxo-1,2,3,4-tétrahydroquinolin-6-yl)-acétamide

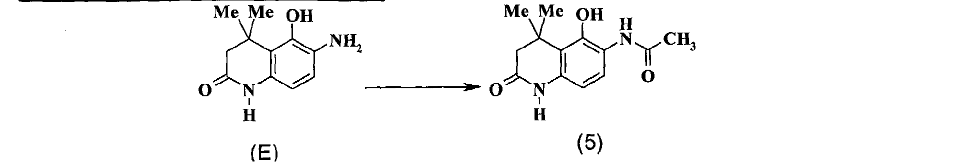

En conservant la température en dessous de 5°C, 8,1 ml de chlorure d'acétyle sont ajoutés lentement à un mélange contenant du diméthylacétamide (50 ml), de l'acétate d'éthyle (50 ml) et 19 g de composé (E). Le mélange de réaction est ensuite mélangé à de l'eau glacée et agité. Le produit ainsi obtenu est filtré. Après séchage et lavage dans 100 ml d'acétate d'éthyle chaud, 22,5 g de produit (5) sont isolés par filtration et séchés.
RMN 1H (300 MHz), CDCl3) : δ 1,32 (6H , s) 2,09 (3H , s) 2,31 (2H , s) 6, 37 (2H , d) 6,88 (2H , d) 9,36 (1H , s) 9,89(1H, brs) 10,00 (1H , b r s)
MS m /z 247 (M+)
Point de fusion = 211-212 °C

### Exemple 6 : synthèse du N-(8-méthoxy-5-hydroxy-4,4-diméthyl-2-oxo-1,2,3,4-tétrahydroquinolin-6-yl)-acétamide

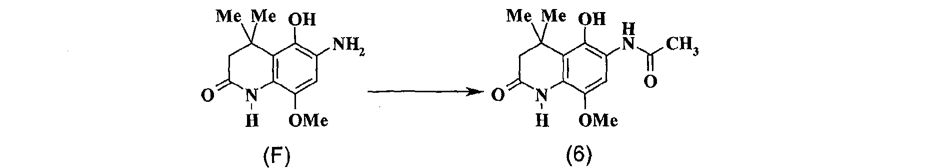

Le composé (F) est préparé selon la synthèse décrite dans le brevet US 4 430 423. En conservant la température en dessous de 5°C, 4,2 ml de chlorure d'acétyle sont ajoutés lentement à un mélange contenant du diméthylacétamide (20 ml), de l'acétate d'éthyle (30 ml) et 10 g de composé (F). Le mélange de réaction est ensuite mélangé à de l'eau glacée et agité. Le produit ainsi obtenu est filtré. Après séchage et lavage dans 100 ml d'acétate d'éthyle chaud, 10,7 g de produit (6) sont isolés par filtration et séchés.
RMN 1H (300 MHz), CDCl3) : δ 1,37 (6H , s) 2,10 (3H , s) 2,31 (2H , s) 3,79 (3H , s)
6,72 (1H, s) 8,80 (1H , s) 8,88 (1H, b r s) 9,90 (1H , b r s)
MS m /z 277 (M+)
Point de fusion = 176-178 °C

### Exemples de teintures

Les compositions tinctoriales suivantes ont été réalisées :

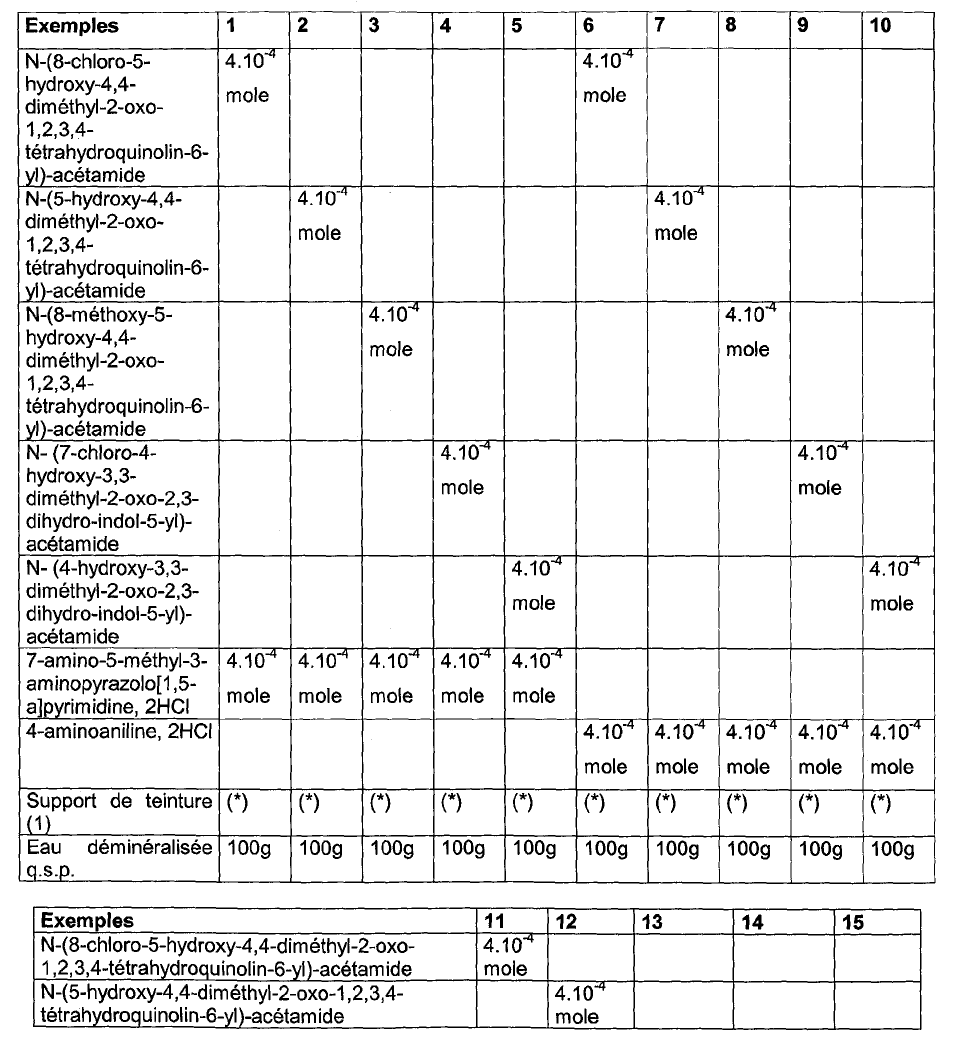

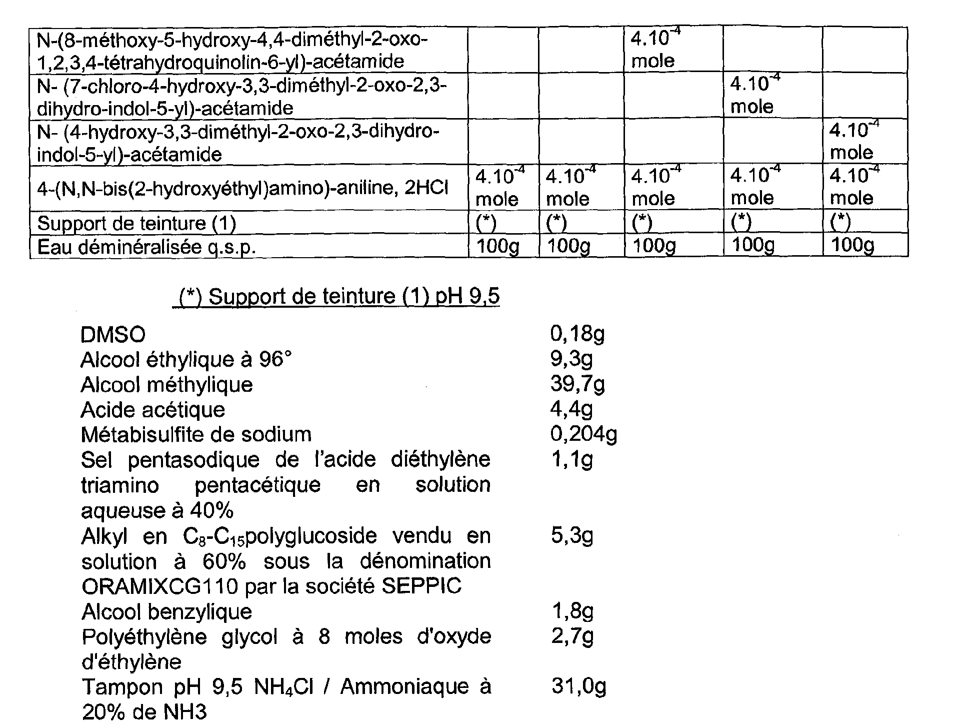

Au moment de l'emploi, la composition est mélangée avec un tiers de son poids d'eau oxygénée à 20 volumes (6% en poids).

Le mélange obtenu est appliqué sur une mèche de cheveux gris à 90 % de blancs. Après 30 min de pose, la mèche est rincée, lavée avec un shampooing standard, rincée à nouveau puis séchée.

Nuances observées :

| **Exemples** | **1** | **2** | **3** | **4** | **5** | **6** | **7** | **8** |
|---|---|---|---|---|---|---|---|---|
| **Nuance** **observée** | Rouge violacé | Rouge violacé | Rouge violacé | violet | violet | Bleu | Bleu | Bleu |
| **Exemples** | **9** | **10** | **11** | **12** | **13** | **14** | **15** | |
| **Nuance** **observée** | violacé | violacé | Bleu | Bleu | Bleu | Vert bleuté | Vert | |

## Revendications

1. Composition tinctoriale comprenant, dans un milieu cosmétique approprié à la teinture des fibres kératiniques, au moins une base d'oxydation et au moins un coupleur de formule (I) suivante et/ou un de ces sels d'addition avec un acide ou une base 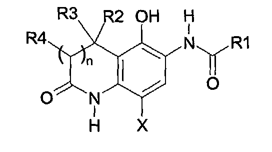 dans laquelle
• **R**_{**1**} est un radical alkyle comprenant de 1 à 10 atomes de carbone, le radical alkyle étant éventuellement substitué par un ou plusieurs radicaux choisis parmi les atomes d'halogène, les radicaux hydroxy, alcoxy, carboxy, alkyl(C₁-C₄)carboxamido, sulfonamido, alkyl(C₁-C₄)sulfonamido, NR₁₁,R₁₂, R₁₁ et R₁₂, représentant indépendamment un atome d'hydrogène, un radical (C₁-C₂)alkyle pouvant être substitué par un hydroxy ou un (C₁-C₂)alcoxy;
• **R**_{**2**}**, R**_{**3**} et **R**_{**4**} représentent indépendamment
(i) un atome d'hydrogène,
(ii) un radical (C₁-C₄)alkyle éventuellement substitué par un ou plusieurs radicaux choisis parmi les radicaux hydroxy, alcoxy, carboxy, alkyl(C₁-C₄)carboxamido (AlkNHCO-), alkyl(C₁-C₄)sulfonyle (AlkSO₂-), alkyl(C₁-C₄)sulfoxyde (AlkylSO-), sulfonamido (NH2SO2-), alkyl(C₁-C₄)sulfonamido (AlkylNHSO2-) ou sulfonique (-SO₃H) ou NR₁₁R₁₂,
• **X** représente un atome d'hydrogène, un atome d'halogène, un radical (C₁-C₄)alcoxy radical, un radical aryloxy radical, ces radicaux pouvant être substitués, et
• n est 0 ou 1.

2. Composition selon la revendication 1 dans laquelle R₁ représente un radical alkyle en C₁-C₆.

3. Composition selon la revendication 1 ou 2 dans laquelle R₁ représente un radical alkyle non substitué.

4. Composition selon l'une quelconque des revendications 1 à 3 dans laquelle R₂, R₃ et R₄ représentent indépendamment un atome d'hydrogène, un radical alkyle en C₁-C₄ éventuellement substitué par un ou plusieurs radicaux choisis parmi les radicaux hydroxy, méthoxy, éthoxy, carboxy, alkyl(C1-C4)carboxamido, sulfonamido ou NR₁₁R₁₂.

5. Composition selon la revendication 4 dans laquelle R₂, R₃ et R₄ sont choisis parmi un atome d'hydrogène, un radical alkyle non substitué.

6. Composition selon l'une quelconque des revendications précédentes dans laquelle X représente un atome d'hydrogène, un atome de chlore, un radical alcoxy.

7. Composition selon l'une quelconque des revendications précédentes dans laquelle le coupleur correspond à la formule (I') 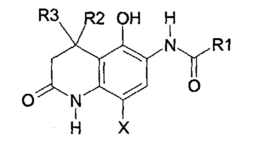 dans laquelle R₁ représente un radical alkyle en C₁-C₆, X représente un atome d'hydrogène, un atome d'halogène ou un radical alcoxy, et R₂ et R₃ représentent un radical méthyle ou éthyle.

8. Composition selon l'une quelconque des revendications précédentes dans laquelle le coupleur de formule (1) ou (I') est choisi parmi les composés :
N-(8-chloro-5-hydroxy-4,4-diméthyl-2-oxo-1,2, 3,4-tétrahydroquinolin-6-yl)-acétamide
N-(5-hydroxy-4,4-diméthyl-2-oxo-1,2, 3,4-tétrahydroquinolin-6-yl)-acétamide
N-(8-chloro-5-hydroxy-4,4-diméthyl-2-oxo-1,2,3,4-tétrahydroquinolin-6-yl)-propylamide
N-(5-hydroxy-4,4-diméthyl-2-oxo-1, 2,3,4-tétrahydroquinolin-6-yl)-propylamide
N-(8-méthoxy-5-hydroxy-4,4-diméthyl-2-oxo-1, 2,3,4-tétrahydroquinolin-6-yl)-acétamide
N-(8- méthoxy -5-hydroxy-4,4-diméthyl-2-oxo-1,2,3,4-tétrahydroquinolin-6-yl)-propylamide
N-(8-chloro-5-hydroxy-2-oxo-1,2,3,4-tétrahydroquinolin-6-yl)-acétamide
N-(5-hydroxy-2-oxo-1,2,3,4-tétrahydroquinolin-6-yl)-acétamide
N-(8-chloro-5-hydroxy-2-oxo-1,2,3,4-tétrahydroquinolin-6-yl)-propylamide
N-(5-hydroxy- 2-oxo-1,2,3,4-tétrahydroquinolin-6-yl)-propylamide
N-(8-méthoxy-5-hydroxy-2-oxo-1,2,3,4-tétrahydroquinolin-6-yl)-acétamide
N-(8-méthoxy -5-hydroxy-2-oxo-1,2,3,4-tétrahydroquinolin-6-yl)-propylamide
N-(7-chloro-4-hydroxy-3,3-diméthyl-2-oxo-2,3-dihydro-indol-5-yl)-acétamide
N- (7-méthoxy-4-hydroxy-3,3-diméthyl-2-oxo-2,3-dihydro-indol-5-yl)-acétamide
N- (4-hydroxy-3,3-diméthyl-2-oxo-2,3-dihydro-indol-5-yl)-acétamide
N- (7-chloro-4-hydroxy-3,3-diméthyl-2-oxo-2,3-dihydro-indol-5-yl)-propylamide
N- (7-méthoxy-4-hydroxy-3,3-diméthyl-2-oxo-2,3-dihydro-indol-5-yl)- propylamide
N- (4-hydroxy-3,3-diméthyl-2-oxo-2,3-dihydro-indol-5-yl)- propylamide
N- (7-chloro-4-hydroxy-2-oxo-2,3-dihydro-indol-5-yl)-acétamide
N- (7-méthoxy-4-hydroxy-2-oxo-2,3-dihydro-indol-5-yl)-acétamide
N- (4-hydroxy-2-oxo-2,3-dihydro-indol-5-yl)-acétamide
N- (7-chloro-4-hydroxy-2-oxo-2,3-dihydro-indol-5-yl)-propylamide
N- (7-méthoxy-4-hydroxy-2-oxo-2,3-dihydro-indol-5-yl)- propylamide
N- (4-hydroxy-2-oxo-2,3-dihydro-indol-5-yl)- propylamide

9. Composition selon la revendication 1 dans laquelle le coupleur acylaminophénol de formule (1) est choisi parmi le N-(8-chloro-5-hydroxy-4,4-diméthyl-2-oxo-1,2,3,4-tétrahydroquinolin-6-yl)-acétamide, le N-(5-hydroxy-4,4-diméthyl-2-oxo-1,2,3,4-tétrahydroquinolin-6-yl)-acétamide, le N-(8-chloro-5-hydroxy-4,4-diméthyl-2-oxo-1,2,3,4-tétrahydroquinolin-6-yl)-propylamide, le N-(5-hydroxy-4,4-diméthyl-2-oxo-1,2,3,4-tétrahydroquinolin-6-yl)-propylamide, le N-(8-méthoxy-5-hydroxy-4,4-diméthyl-2-oxo-1,2,3,4-tétrahydroquinolin-6-yl)-acétamide, le N-(8- méthoxy -5-hydroxy-4,4-diméthyl-2-oxo-1,2,3,4-tétrahydroquinolin-6-yl)-propylamide, le N-(8-chloro-5-hydroxy-2-oxo-1,2,3,4-tétrahydroquinolin-6-yl)-acétamide, le N-(5-hydroxy-2-oxo-1,2,3,4-tétrahydroquinolin-6-yl)-acétamide, le N-(8-chloro-5-hydroxy-2-oxo-1,2,3,4-tétrahydroquinolin-6-yl)-propylamide, le N-(5-hydroxy- 2-oxo-1,2,3,4-tétrahydroquinolin-6-yl)-propylamide, le N-(8-méthoxy-5-hydroxy-2-oxo-1,2,3,4-tétrahydroquinolin-6-yl)-acétamide, le N-(8- méthoxy -5-hydroxy-2-oxo-1,2,3,4-tétrahydroquinolin-6-yl)-propylamide.

10. Composition selon l'une quelconque des revendications 1 à 9 dans laquelle la quantité de chacun des coupleurs est comprise entre 0,001 et 10 % en poids environ du poids total de la composition tinctoriale.

11. Composition selon l'une quelconque des revendications précédentes dans laquelle la base d'oxydation est choisie parmi les paraphénylènediamines, les bis-phénylalkylènediamines, les para-aminophénols, les bis-paraaminophénols, les ortho-aminophénols, les bases hétérocycliques et leurs sels d'addition.

12. Composition selon la revendication 11 dans laquelle la quantité de chacune des bases est comprise entre 0,001 et 10 % en poids environ du poids total de la composition tinctoriale.

13. Procédé de teinture d'oxydation des fibres kératiniques, **caractérisé en ce qu'**on applique sur les fibres une composition tinctoriale telle que définie dans l'une quelconque des revendications 1 à 12 en présence d'un agent oxydant pendant un temps suffisant pour développer la coloration désirée.

14. Procédé selon la revendication 13 dans lequel l'agent oxydant est choisi parmi le peroxyde d'hydrogène, le peroxyde d'urée, les bromates de métaux alcalins, les persels, les peracides et les enzymes oxydases.

15. Dispositif à plusieurs compartiments dans lequel un premier compartiment contient une composition tinctoriale telle que définie à l'une quelconque des revendications 1 à 12 et un deuxième compartiment contient un agent oxydant.

16. Kit de teinture des fibres kératiniques comprenant une première composition tinctoriale telle que définie selon l'une quelconque des revendications 1 à 12 et une deuxième composition comprenant un agent oxydant.

17. Utilisation du composé de formule (I) tel que défini aux revendications 1 à 10 pour la teinture de fibres kératiniques.
